# EUROPEAN PATENT SPECIFICATION

(11) **EP 4 271 296 B1**
(45) Date of publication and mention of the grant of the patent: **08.04.2026**
(21) Application number: 21914765.9
(22) Date of filing: 30.12.2021
(51) Int. Cl.: A61B 18/20, A61B 90/00, A61F 7/00, A61B 5/00, A61B 18/22, A61B 18/00

(54) **LASER DEVICE FOR LASER TREATMENT OF SKIN LESIONS UNDER DERMAL VASOCONSTRICTION**
LASERVORRICHTUNG ZUR LASERBEHANDLUNG VON HAUTLÄSIONEN UNTER DERMALER GEFÄSSVERENGUNG
DISPOSITIF LASER POUR LE TRAITEMENT AU LASER DE LÉSIONS CUTANÉES SOUS UNE VASOCONSTRICTION DERMIQUE

(30) Priority: 31.12.2020 KR 20200189833
(43) Date of publication of application: 08.11.2023
(73) Proprietor: CLASSYS INC., Seoul 06220 (KR)
(72) Inventor: KIM, Yonghan, Anyang-si, Gyeonggi-do 14084 (KR); LEE, Sang Joon, Asan-si, Chungcheongnam-do 31450 (KR); LEE, Jae Ho, Suwon-si, Gyeonggi-do 16279 (KR)
(74) Representative: Stöckeler, Ferdinand
(86) International application number: PCT/IB2021/000926
(87) International publication number: WO 2022/144587

(56) References cited:
- KR-B1- 101 997 891
- US-A1- 2002 120 315
- US-A1- 2011 184 396
- US-A1- 2016 081 541
- US-A1- 2017 304 645
- US-A1- 2020 163 714
- US-B1- 10 531 920
- US-B1- 6 485 484
- US-B1- 6 770 069

## Description

### TECHNICAL FIELD

This invention relates to devices for dermatological laser treatment

### BACKGROUND

Laser skin treatments are disclosed e. g. in US 2011/0184396 A1, US 2002/120315 A1 and US 6 770 069 B1. Laser treatment has been widely used in dermatology to treat various vascular and pigmented skin lesions, tattoos, and benign skin tumors. Of these, the skin lesions most frequently treated by the lasers are pigmented skin lesions, such as seborrheic keratosis, melasma, ephelis, solar lentigo, melanocytic nevus, and dermal melanocytosis.

A pigmented skin lesion is caused by an excessive increase in the amount of melanin in the skin. Melanin is biosynthesized in the dendritic cells, called melanocyte, that exist in the stratum basale of the skin. If the amount of melanin is excessive or if it exists in the location where melanin does not exist under a normal condition, such a condition can be diagnosed as a pigmentary skin lesion. At times, such a pigmentary skin lesion can propagate or shrink the epidermis or pathologically alter some elements of the skin, such as various skin appendage of dermis.

Generally, an excessive generation of the amount of melanin in the skin is caused by exposure of the skin to the UV radiation or inflammation of the skin. When melanin is generated excessively or when already-generated melanin moves to the keratinocytes by a stimulus, it can create various pigmentary lesions or aggravate the preexisting pigmentary lesions.

Post-inflammatory hyperpigmentation (PIH) is a reaction of epidermal melanocytes and a variety of dermal cells to a stimulus on the skin. It occurs as a result of abnormal distribution of the existing melanin or increase of melanin in the wounded area. In the end, excess melanin causes excessive hyperpigmentation to occur in the area of inflammation. The duration of hyperpigmentation can be affected by local or systemic factors, and, in some cases, it can last for an extended period of time-from several months to several years.

### SUMMARY

The invention is defined in the appended set of claims. Methods mentioned hereinafter do not form part of the present invention.

A laser device for treating a skin lesion can comprise: (i) a laser source; (ii) a light transmitting member, which includes a top surface that forms a laser transmission surface and a bottom surface that forms a skin contacting surface, allowing a treatment laser beam from the laser source to transmit from the laser transmission surface to the skin contacting surface; (iii) a cooling unit that causes the skin contacting surface of the light transmitting member to cool, thereby allowing vasoconstriction of a treatment site to occur by cooling the treatment site with the skin contacting surface; (iv) a control unit that controls the cooling unit and enables the treatment laser beam to be irradiated on the skin lesion in the treatment site under vasoconstriction by controlling the laser source; and (v) a heater attached to the light transmitting member, where the control unit controls the heater to prevent or delay fogging on the light transmitting member.

In certain embodiments, the laser device can further include a camera that obtains an image of the treatment site that comes into contact with the skin contacting surface. The light transmitting member comprises a first light transmitting portion with its top surface forming said laser transmission surface; and a second light transmitting portion located at the bottom of said first light transmitting portion with its bottom surface forming said skin contact surface, where said second light transmitting portion having a skin contact surface is constructed to have a material with higher thermal conductivity than said first light transmitting portion.

In certain embodiments, said cooling unit can comprise a thermoelectric element and a conductive member with said thermoelectric element attached to one side and the other side thereof attached to said second light transmitting portion, thus cooling said second light transmitting portion by conducting heat with said thermoelectric element.

In certain embodiments, said light transmitting member can comprise: i) a first light transmitting portion forming said transmission surface with its top surface; ii) a second light transmitting portion located at the bottom of said first light transmitting portion to form said skin contacting surface; and iii) a beam splitter located between said first light transmitting portion and said second light transmitting portion to transmit said laser beam and reflects the light reflected from said skin contacting surface to one side; and further comprise a camera that obtains the image of the treatment site that comes into contact with said skin contacting surface from the light reflected from said beam splitter.

In certain embodiments, a light adjustment unit can adjust the target position of the laser beam transmitting through said light transmitting member, where said control unit can specify the location of the skin lesion by analyzing the image obtained by said camera; control said light adjustment unit using the location on said specified skin lesion as the target location; and cause said laser beam to be irradiated on said target location.

In certain embodiments, said conductive member can comprise: a conductive base, attached to the heat absorption plate of said thermoelectric element, to transfer heat; and a conductive bridge to cool off said second light transmitting portion by transferring heat between said conductive base and said second light transmitting portion and form the spaced portion between said conductive base and said second light transmitting portion.

In certain embodiments, an insulation material can be filled in the spaced portion between said conductive base and said second light transmitting portion.

In certain embodiments, said light transmitting member can be constructed with a first light transmitting block and a second light transmitting block with different heat transfer coefficients alternatingly stacked up from said laser transmission surface to said skin contacting surface.

In certain embodiments, at a temperature maintaining means can be further comprised to maintain the temperature of said light transmitting member within a set range to prevent or delay fogging on said light transmitting member by said cooling unit.

In certain embodiments, a heater attached to one side of said light transmitting member can be further comprised to one side of said light transmitting member; and said control unit can control said heater to prevent or delay fogging on said light transmitting member by said cooling unit.

A device for treating a skin lesion can comprise: (i) a laser source; (ii) a light transmitting member, which includes a top surface that forms a laser transmission surface and a bottom surface that forms a skin contacting surface, where the light transmitting member allows a treatment laser beam from the laser source to transmit from the laser transmission surface to the skin contacting surface, where the light transmitting member comprise a first light transmitting block having a first heat transfer coefficient and a second light transmitting block having a second heat transfer coefficient, where the first light transmitting block and the second light transmitting block are alternatingly stacked up from the laser transmission surface to the skin contacting surface; (iii) a cooling unit that causes the skin contacting surface of the light transmitting member to cool, thereby inducing vasoconstriction of a treatment site by cooling the treatment site with the skin contacting surface; and (iv) a control unit that controls the cooling unit and enables the treatment laser beam to be irradiated on the skin lesion in the treatment site under vasoconstriction by controlling the laser source.

In certain embodiments, the laser device can further include a heater attached to the light transmitting member, where the control unit controls the heater to prevent or delay fogging on the light transmitting member.

In certain embodiments, the laser device can further include a camera that obtains an image of the treatment site that comes into contact with the skin contacting surface.

A laser device for treating a skin lesion can comprise: (i) a laser source; (ii) a light transmitting member, which includes a top surface that forms a laser transmission surface and a bottom surface that forms a skin contacting surface, where the light transmitting member allows a treatment laser beam from the laser source to transmit from the laser transmission surface to the skin contacting surface; (iii) a cooling unit that causes the skin contacting surface of the light transmitting member to cool, thereby inducing vasoconstriction of a treatment site by cooling the treatment site with the skin contacting surface; (iv) a control unit that controls the cooling unit and enables the treatment laser beam to be irradiated on the skin lesion in the treatment site under vasoconstriction by controlling the laser source; and (v) a sensor that detects a gap between the skin contacting surface and the treatment site.

In certain embodiments, the sensor can measure an impedance of the treatment site.

In certain embodiments, the laser device can further include a heater attached to the light transmitting member, where the control unit controls the heater to prevent or delay fogging on the light transmitting member.

In certain embodiments, the laser device can further include a camera that obtains an image of the treatment site that comes into contact with the skin contacting surface.

A laser device for treating a skin lesion can comprise: (i) a laser source; (ii) a light transmitting member, which includes a top surface that forms a laser transmission surface and a bottom surface that forms a skin contacting surface, where the light transmitting member allows a treatment laser beam from the laser source to transmit from the laser transmission surface to the skin contacting surface; (iii) a cooling unit that causes the skin contacting surface of the light transmitting member to cool, thereby inducing vasoconstriction of a treatment site by cooling the treatment site with the skin contacting surface; (iv) a camera that obtains an image of the treatment site that comes into contact with the skin contacting surface; (v) a control unit that controls the cooling unit and enables the treatment laser beam to be irradiated on the skin lesion in the treatment site under vasoconstriction by controlling the laser source; and (vi) a scanner that facilitates irradiation of the laser beam to the treatment site based on the image obtained by the camera.

In certain embodiments, the scanner can be a galvanometer scanner.

In certain embodiments, the laser device can further include a heater attached to the light transmitting member, where the control unit controls the heater to prevent or delay fogging on the light transmitting member.

A laser device for treating a skin lesion can comprise: (i) a laser source; (ii) a conductor frame formed of a conductor; (iii) a light transmitting member, which includes a first window that forms a laser transmission surface on the top surface of the conductor frame, a second window that forms a skin contacting surface on the bottom surface of the conductor frame, and a light transmission gas sealed inside the conductor frame; where the light transmitting member allows a treatment laser beam from the laser source to transmit from the laser transmission surface to the skin contacting surface; (iv) a cooling unit that causes the skin contacting surface of the light transmitting member to cool, thereby inducing vasoconstriction of a treatment site by cooling the treatment site with the skin contacting surface; (v) a control unit that controls the cooling unit and enables the treatment laser beam to be irradiated on the skin lesion in the treatment site under vasoconstriction by controlling the laser source; and (vi) a heater attached to the light transmitting member, where the control unit controls the heater to prevent or delay fogging on the light transmitting member.

In certain embodiments, the laser device can further include a beam splitter located inside the conductor frame and a third window located on one side of the conductor frame, where the beam splitter sends an image of the skin lesion to a camera via the third window.

In certain embodiments, the laser device can further include a camera that obtains an image of the treatment site that comes into contact with the skin contacting surface.

In certain embodiments, the laser device for laser treatment of skin lesion under dermal vasoconstriction can induce vasoconstriction with a cooling device to minimize inflammation triggered as a result of irradiating laser on the normal tissue and the capillary loops; and brings about an effect that the post-laser treatment adverse effects such as hyperpigmentation, erythema, etc., can be minimized by performing laser treatment on the skin lesions under vasoconstriction.

In certain embodiments, the present invention can have an effect of minimizing the adverse effects of laser treatments by obtaining and analyzing images of the specific skin lesions under vasoconstriction and enable to complete laser treatment promptly and accurately, targeting the specific lesions.

In certain embodiments, the present invention can prevent or significantly delay occurrence of fogging onto the light transmitting member caused by a cooling device and thereby stably collect the images of lesions; thus, it can have an effect of performing stable laser treatments using said images.

A method of treating a skin lesion using a laser device can include irradiating the treatment laser beam in an overlapping manner. In certain embodiments, the overlapping manner can be determined by a size of the treatment laser beam, an overlapping percentage, an area of the treatment site, and a resolution of the image obtained by the camera. The skin lesion can be treated with laser light under vasoconstriction so that damages caused by the laser energy can be minimized for the overlapping area.

A method of treating a skin lesion can include irradiating the laser light on a skin lesion beyond the boundary of the skin lesion identified on the skin surface. The skin lesion can be treated with laser light under vasoconstriction so that damages caused by the laser energy can be minimized for the treated area.

### BRIEF DESCRIPTION OF THE DRAWINGS

FIG. 1A is a drawing of an exemplary embodiment of the laser device. FIG. 1B is a drawing of the laser device, illustrated in FIG. 1A, viewed from the side.
FIG. 2 is a drawing of another exemplary embodiment of the laser device.
FIG. 3A is a schematic image captured by the camera of the laser device. FIG. 3B shows how the captured image can be processed and analyzed. FIG. 3C shows how the boundary of the skin lesion area can be specified. FIG. 3D shows how the laser beam can be irradiated targeting the locations within the boundary, forming multiple beam spots within the boundary after specifying the boundary of the skin lesion area. FIG. 3E shows that the laser beam can be irradiated in an overlapping manner between multiple beam spots. FIG. 3F shows a type of handling after the laser treatment.
FIG. 4 is a drawing of another exemplary embodiment of the laser device.
FIG. 5A is a drawing of an exemplary embodiment of the improved construction of the cooling unit to solve the problem of fogging on the laser device. FIG. 5B is a drawing of yet another exemplary embodiment of the improved construction of the cooling unit to solve the problem of fogging on the laser device.
FIG. 6A is a drawing of an exemplary embodiment of the improved construction of the cooling unit to solve the problem of fogging on the laser device. FIG. 6B is a drawing of yet another exemplary embodiment of the improved construction of the cooling unit to solve the problem of fogging on the laser device.
FIG. 7A is a drawing of an exemplary embodiment of the laser device. FIG. 7B is a plan view of the laser device illustrated in FIG. 7A. FIG. 7C is a bottom view of the laser device illustrated in FIG. 7A.
FIG. 8 shows an embodiment of the hand piece of the laser device implemented with a touch sensor.
FIG. 9A illustrates an exemplary skin lesion identified by the computer algorithm. FIG. 8B illustrates an example of the area a practitioner intends to treat for the same skin lesion as FIG. 9A.
FIG. 10 is an exemplary embodiment of the hand piece including a scanner.
FIG. 11A is an image of a skin lesion whose boundary is marked with a dotted line using a galvanometer scanner. FIG. 11B is an image transformed using the threshold image processing method. FIG. 11C is an image where the internal area connected by the dots is designated for laser irradiation by adjusting the threshold value.
FIG. 12A is an image of a skin lesion obtained by a camera, in which the area of the skin lesion is marked with a dotted line using a galvanometer scanner. FIG. 12B shows the same image as FIG. 12A with the hue saturation value. FIG. 12C shows the same image as FIG. 12A, in which the area of the skin lesion is filled using an equalized histogram.
FIG. 13A is the same image as FIG. 12A transformed using a threshold image processing method to extract the area marked by the galvanometer scanner. FIG. 13B shows the same image as FIG. 13A with the threshold hue saturation value. FIG. 13C shows the same image as FIG. 13A with the internal area connected by the dots is filled using an threshold equalized histogram.
FIG. 14 shows that there can be an area where the laser light is not irradiated depending on the size of the laser spot.
FIG. 15A shows an exemplary irradiation pattern with an overlapping percentage value of 20%. FIG. 15B shows an exemplary irradiation pattern with an overlapping percentage value of 40%. FIG. 15C shows an exemplary irradiation pattern with an overlapping percentage value of 50%.
FIG. 16 shows an exemplary circumstance where the laser can be irradiated on a normal skin area surrounding the skin lesion.

### DETAILED DESCRIPTION

Due to the recent advancement of laser technology, laser equipment for treating skin lesions is being developed in various ways. Skin lesions can be treated using high power energy of a laser to selectively destroy target chromophores in the epidermis and/or dermis of the skin, while minimizing skin damages by shortening the duration of exposure of the skin to the laser energy.

While laser treatments for skin lesions are generally considered safe and effective, they can lead to various adverse reactions depending on the type of treatment procedures.

Immediately following a laser treatment, adverse reactions, such as blisters, scabs, bacterial infections, and hypersensitivity reaction to anesthetic ointment, can occur. Prolonged post-treatment adverse reactions, such as erythema, hyperpigmentation, and scars, can occur as well.

Post-laser treatment reactions can be caused by localized inflammatory reactions triggered by damages to numerous cells residing in the skin. Damaged capillary loops located in the papillary dermis and resulting migration of the inflammation cells in the blood vessels and continued inflammatory reactions by the migrated cells can lead to adverse reactions, such as post-inflammatory hyperpigmentation (PIH) and post-laser erythema (PLE).

There has been no clinical satisfactory method to reduce adverse reactions caused by laser treatment when treating skin lesions. Damages to the capillary loops have been attempted to be reduced by using local anesthetics or injected anesthetics in conjunction with the laser treatment, but it has not been effective.

Disclosed herein is a laser device for treating a skin lesion, specifically, a laser device that can temporarily constrict the blood vessels under the treatment site to induce vasoconstriction when treating various pigmented and non-pigmented skin lesions, such as seborrheic keratosis, melasma, ephelis, nevus, vascular skin disorders, and skin cancers.

In certain embodiments, the laser device can induce vasoconstriction with a cooling device to minimize inflammation triggered by the irradiation of the laser beam on the normal skin tissue and the capillary loops and to minimize post-laser treatment adverse reactions, such as hyperpigmentation and erythema.

In certain embodiments, the laser device can minimize the adverse reactions of the laser treatments by obtaining and analyzing images of a specific skin lesion under vasoconstriction and enables to complete laser treatment promptly and accurately, by targeting the specific lesion.

A wavelength of the laser can be selected in accordance with the target chromophore for the laser treatment to selectively destroy only the chromophore that absorbs the particular wavelength of the light-hence, this technique is called selective photothermolysis (SPTL). However, in reality, any wavelength of the laser can be absorbed by any target to a certain degree, thereby causing unwanted reactions in the skin.

For example, when a laser for a pigmentary lesion is applied to melanin as a target chromophore, not only the melanin-producing melanocytes, but also the keratinocytes, which forms most of the epidermis, can be damaged by the laser. This is because a lot of melanin pigments are constantly being delivered to keratinocytes across multiple layers of epidermis via melanosomes. When keratinocytes are damaged, these cells can secrete inflammatory cytokines and induce various adverse reactions.

Various conditions and factors can affect the effectiveness and precision of SPTL, such as the diameter of the beam, fluence, pulse duration, and stacking/overlapping of the laser beam spots, as well as the unique characteristics of the laser beam wavelength itself.

In a conventional laser treatment, when the fluence is raised higher than a conventional level or the laser beam spots are overlapped with one another to apply excessive laser energy, inflammatory skin reactions, such as petechial hemorrhage and dermal edema, can appear regardless of the type of laser. Accordingly, various adverse reactions can follow after the laser treatment, such as post-inflammation hyperpigmentation (PIH), persistent post-inflammatory erythema (PIE), scars, and textural deformity.

Intense inflammatory reactions caused by the petechial hemorrhage and the dermal edema can be especially more conspicuous with the Q-switched 532 nm laser, among various Q-switched lasers. This is because this particular wavelength of the laser light can be absorbed not only by the melanin pigments but also by the hemoglobin, which can cause severe inflammatory reactions even at a low energy level which would not normally cause much damages to the capillary loops of the papillary dermis by other lasers.

Any laser used for the skin treatment can damage the capillary loops located in the papillary dermis to a certain degree. As the fluence is increased, the degree of damages to the capillary loops also increases accordingly. Hence, to obtain a desired treatment effect while minimizing the adverse reactions of the skin caused by the laser irradiation, it is desirable to preserve the capillary loops as much as possible.

To preserve the capillary loops, it is important to consider that the target chromophore of the capillary loops reacting to the laser is not the wall of the blood vessels of the capillary loops, but the hemoglobin (oxy/deoxy Hb, "the moving chromophore") in the red blood cells that moves around in the blood vessels. Thus, if the amount of red blood cells flowing inside the capillary loops can be temporarily reduced during the laser treatment time, the amount of the target chromophore reacting to the laser light can be decreased, and the effect of the laser energy on the capillary loops via red blood cells can be reduced.

To minimize the damages to the capillary loops caused by the laser energy, the area of the skin lesion can be artificially cooled immediately prior to and during the laser treatment. The autonomic nervous system then reacts immediately to maintain the body temperature. As a result, the precapillary sphincter, which delivers the blood to the capillary loops of the papillary dermis, can be temporarily constricted, allowing the blood to flow only to the sphincter superficial vascular plexus. **In** other words, the area of the skin lesion is under vasoconstriction in this state.

If the amount of red blood cells decreases in the capillary loops of the papillary dermis under vasoconstriction, the target chromophore (i.e. hemoglobin) which reacts to the laser decreases, and the damages to the wall of capillary loops can be reduced. As a result, the amount of inflammatory cells, which migrate to the area through the blood vessels can be decreased, which means that the inflammatory reactions in the area on which the laser light is irradiated can be significantly reduced.

If the blood vessels and tissues around papillary dermis can be preserved to minimize damages, normal wound healing processes can be induced more quickly.

As described above, the laser device disclosed herein can be constructed such that the blood vessels are contracted (i.e. "under vasoconstriction") as the area of the skin lesion is artificially cooled immediately before and during laser treatment. As a result, the amount of red blood cells in the capillary loops of the papillary dermis can decrease, and the laser beam can be irradiated to treat the skin lesion while the amount of the hemoglobin is reduced in the treatment site. The treatment using this device can decrease the adverse reactions of the laser treatment, such as hyperpigmentation, bums, erythema, and scars, and can be applied to any pigmented and non-pigmented skin lesions, including hard-to-treat skin lesions, such as solar lentigo, nevus of Ota, bilateral nevus of Ota macule, and large congenital melanocytic nevus.

If the laser treatment is applied under vasoconstriction, skin lesions in dermis can be treated without causing hyperpigmentation or damaging the epidermis. The number of treatments and the time duration required for full recovery can be reduced by increasing the intensity of the laser power, and accordingly, excellent treatment results can be obtained. The fluence can be increased far higher than the conventional intensity, which makes it possible to treat without serious adverse reactions: (i) light-colored depigmented lesions with relatively small degree of melanin hyperpigmentation as a type of epidermis pigmentation lesions; (ii) seborrheic keratosis, with minutely thick lesions, caused by clonal proliferation; and (iii) solar lentigo.

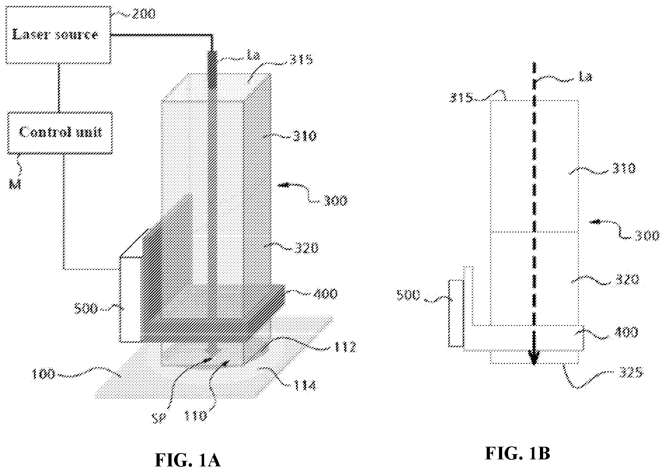

FIGS. 1A and 1B illustrate an exemplary embodiment of the laser device for treating skin lesions under dermal vasoconstriction. FIG. 1A is a drawing of an exemplary embodiment of the laser device. FIG. 1B is a drawing of the laser device illustrated in FIG. 1A, viewed from the side.

As illustrated in FIG. 1A and FIG. 1B, an exemplary embodiment can comprise: a laser source **200,** a light transmitting member **300,** cooling unit **400** and **500,** and a control unit **M.** The laser source **200** is a component that oscillates the laser to treat skin lesions, such as non-pigmented skin lesions and pigmented skin lesions, as described above. The laser source **200** can comprise lasers, such as ruby, alexandrite, and Nd:YAG lasers. Preferably, it can comprise an Nd:YAG laser that can oscillate two wavelengths for treatment of pigmented skin lesions in dermis and epidermis, caused by melanin pigments.

The light transmitting member **300** is a component that guides the laser beam to be transmitted from the top surface to the bottom surface, as illustrated in FIG. 1A and FIG. 1B. The light transmitting member **300** can include a top surface forming a laser transmission surface **315** and a bottom surface forming a skin contact surface **325** that comes into contact with the lesion **110** of the skin **100.** The light transmitting member **300** can guide the treatment laser beam from the laser source **200** to transmit from the laser transmission surface **315** to the skin contact surface **325,** allowing the laser beam spot (SP) to be formed at the target location of the lesion **110.**

The light transmitting member **300** can comprise a first light transmitting portion **310** and a second transmitting portion **320,** as illustrated in FIG. 1A and FIG. 1B, with the top surface of the first light transmitting portion **310** forming a laser transmission surface **315** and the bottom surface of the second light transmitting portion **320** forming a skin contact surface **325.**

The first light transmitting portion **310** and the second light transmitting portion **320** can be formed of optically transparent materials that can transmit light, such as quartz, sapphire, crystal, poly (methyl methacrylate), or polystyrene. The first light transmitting portion **310** and the second light transmitting portion 320 can comprise different materials from each other; and the second light transmitting portion **320** with a skin contact surface **325** can have a material with a higher thermal conductivity than the first light transmitting portion **310.**

The first light transmitting portion **310** and second light transmitting portion **320** can be formed to adhere to each other by an adhesive, such as UV adhesive.

The cooling unit **400** and **500** can cool down the skin contact surface **325** of the light transmitting member **300** and cause the blood vessel beneath the contact area **110** of the skin **100** to be constricted by cooling down the contact area **110** of the skin **100** with the skin contact surface **325.**

Various types of the cooling unit can be used to cool down the light transmitting member **300.** Non-limiting examples of the cooling unit can include a thermoelectric element, cooling air, cooling gas, or cooling liquid. Preferably, the cooling unit can include a thermoelectric element **500** and a conductive member **400** including a heat conducting material such as metal, as illustrated in FIG. 1A and FIG. 1B.

The thermoelectric element **500** is a component that has a heat absorption plate and a heat radiation plate contacting each other to cool down the heat absorption plate and heat up the heat radiation plate as directed by electric signals. As illustrated in FIG. 1A and FIG. 1B, the heat absorbing side of the thermoelectric element **500** can be attached to one side of the conductive member **400** and the other side of the conductive member **400** is attached to the second light transmitting portion **320.** As a result, the second light transmitting portion **320** can be cooled down while the heat of the second light transmitting portion **320** is transferred to the thermoelectric element **500** through the conductive member **400** as the thermoelectric element **500** absorbs the heat.

As shown in FIG. 1A, when the skin contact surface **325** is cooled down while the skin contact surface **325** of the second light transmitting portion **320** is in contact with the skin contact area **110,** the contacted area becomes the cooling area **112** and its surrounding periphery becomes an area **114** with a slightly higher temperature.

As the thermoelectric element **500** operates, the second light transmitting portion **320** cools down and, as the skin contact surface **325** of the second light transmitting portion **320** cools down, the cooling area **112** of the skin cools down, and the blood vessels underneath said cooling area **112** becomes constricted, and, as a result, the blood flow decreases while the cooling is maintained. If the laser treatment is performed under this condition, the adverse reactions such as hyperpigmentation and erythema can be minimized.

The light transmitting member **300** can be constructed by adhering the first light transmitting portion **310** and the second light transmitting portion **320.** The second light transmitting portion **320** forming the skin contact surface **325** can comprise a material with a higher thermal conductivity than the first light transmitting portion **310** to facilitate cooling of the skin contact surface **325** of the second light transmitting portion **320** with the cooling unit **400** and **500** connected thereto.

In certain embodiments, as illustrated in FIGS. 1A and 1B, the laser device can comprise a control unit **M,** and the control unit **M** can be connected to the laser source **200** and the cooling unit **400** and **500** and control them.

While the skin contact surface **325** of the light transmitting member **320** is being in contact with the skin **100,** the control unit **M** can cool down the skin contact surface **325** by controlling the thermoelectric element **500** of the cooling unit and allow the blood vessels of the cooling area **112** at the contact area **110** of the skin, which comes into contact therewith, to constrict. When it cools down sufficiently for the treatment, the laser source **200** is controlled while the cooling lasts so as the treatment laser beam to be irradiated on the skin contact area under the state of vasoconstriction.

As illustrated in FIG. 1A, while maintaining the cooling of the skin contact surface **325** by using the cooling unit (while maintaining the state of vasoconstriction) in the state in which the skin contact surface **325** of the light transmitting member **300** remains in contact with the contact area **110** of the skin, laser beam **La** transmits light through the light transmitting member **300** to form a spot **SP** on the contact area **110** which is in contact with the skin contact surface. Accordingly, the skin lesion can be treated with the laser under vasoconstriction.

When the skin contact surface **325** of the light transmitting member **300** is being in contact with the contact area on the skin, adverse reactions from overcooling of skin can also be prevented or minimized if it is possible to know to what extent the contact area of said skin is cooled down.

To achieve this, the laser device can separately have a temperature sensor (not shown) for sensing to what extent the contact area **110** of the skin that comes into contact with the skin contact surface **325** of the light transmitting member **300** is cooled down, when the cooling is performed by the cooling unit.

In certain embodiments, the temperature sensor can directly sense the temperature of the skin contact area **110.** In certain other embodiments, the temperature sensor can measure the temperature of the light transmitting member **300** (e.g., the temperature of the second light transmitting portion **320**) and calculate the temperature of the skin contact area based on the temperature information thereof.

By employing the method above, the control unit allows the laser treatment to be performed effectively under vasoconstriction without adverse reactions by controlling the cooling unit, the laser source, etc., based on the temperature information of the skin contact area obtained from the temperature sensor.

When the cooling is performed on the contact area of the skin via the skin contact surface **325** of the light transmitting member **300,** serious adverse reactions can occur from the cooling itself if the cooling time becomes excessively long. Hence, it is important to shorten the duration of the cooling and to perform laser treatment of lesions quickly.

To treat the skin lesion quickly, it is necessary to find the location of the skin lesion on the skin contact area **110** where the skin contact surface **325** of the light transmitting member **300** is contacted and to guide the laser beam to precisely form a spot on the skin lesion.

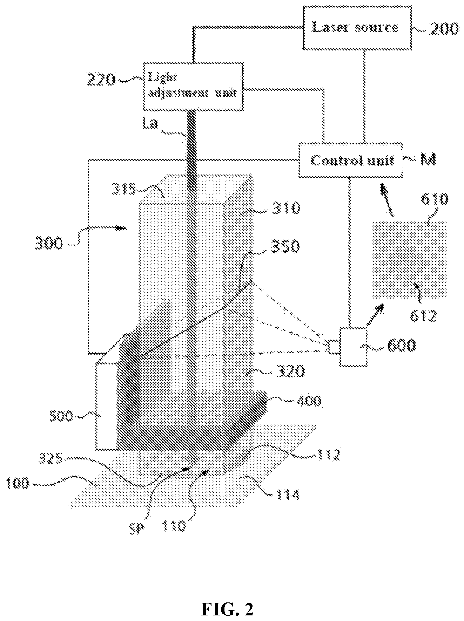

As illustrated in FIG. 2, a laser device can be constructed to find the location of the lesion by analyzing the images of the area of the skin lesion, to target the lesion based on the analysis, and thereby to perform laser treatment quickly and precisely.

In certain embodiments, the laser device can be constructed to comprise: (i) a laser source **200,** a light transmitting member **300,** and cooling unit **400** and **500** in the same manner as the laser device according to an embodiment illustrated in FIGS. 1A and 1B above; additionally, (ii) light adjustment unit **220,** a beam splitter **350,** and a camera **600;** and (iii) a control unit **M** that controls these components.

The laser device according to this embodiment, just like the laser device according to the embodiment illustrated in FIGS. 1A and 1B described above, can comprise: a laser source **200;** a light transmitting member **300,** which includes a top surface forming a laser transmission surface **315** and a bottom surface forming a skin contact surface **325** that contacts the lesion **110** of the skin **100,** and guides the treatment laser beam **La** from the laser source **200** to be transmitted from the laser transmission surface **315** to the skin contact surface **325;** and the cooling unit **400** and **500** that can cool down the skin contact surface **325** of the light transmitting member **300** to induce vasoconstriction by cooling down the skin contact area **110** with the skin contact surface **325.**

The laser source **200,** the light transmitting member **300,** and the cooling unit **400** and **500** can be substantially identical to the laser device according to one embodiment illustrated in FIGS. 1A and 1B.

As illustrated in FIG. **2****,** the light transmitting member **300** can comprise the first light transmitting portion **310** with its top surface forming the laser transmission surface **315;** and the second light transmitting portion **320** with its bottom surface forming the skin contact surface **325,** thereby guiding the laser beam to be transmitted and proceed from the laser transmission surface **315** to the skin contact surface **325.**

The light transmitting member **300** according to this embodiment, as illustrated in FIG. 2, can comprise a beam splitter **350** between the first light transmitting portion **310** and the second light transmitting portion **320;** thereby transmitting the laser beam **La** that proceeds from the laser transmission surface **315** to the skin contact surface **325,** and reflecting to one side the light which is reflected from the contact portion **110** with the skin contact surface **325** contacted thereto.

The camera **600** can capture images of the skin contact area **110** that contacts the skin contact surface from the light reflected from the beam splitter **350.**

To obtain images captured by the camera **600,** it is preferable to use a separate dedicated illumination apparatus, rather than using the natural light. With the illumination apparatus separately provided for the skin contact area, the light reflected from the skin contact area can be reflected on the beam splitter **350,** allowing the camera **600** to capture clearer images of the contact area by receiving the light reflected by the beam splitter **350.** In other words, it is possible to obtain the images of the contact area, as indicated by the image **610** in in FIG. 2.

In certain embodiments, the image **610** of the contact area captured by the camera **600** can be sent to the control unit **M.** The control unit **M** can then analyze the image **610,** specify the lesion **612,** specify the location of the skin lesion **612,** control the light adjustment unit **220** targeting a location on the specified skin lesion, and guide the laser beam to be irradiated on the targeted location.

The light adjustment unit **220** can be a device that allows a beam spot **SP** to be formed on the targeted location of the skin contact area **110** by adjusting the path of the laser beam oscillated from the laser source **200.** In certain embodiments, the light adjustment unit **220** can be a Galbano mirror.

By processing and analyzing the images **610** captured by the camera **600,** the control unit **M** can detect the skin lesion **612** area, specify the boundaries, calculate the locational coordinates of the specified lesion area within the boundary as the coordinate information of the targeted location of the laser beam, control the light adjustment unit **220** in accordance with the coordinate information of the targeted location, and guide the laser beam **La** to be irradiated on the calculated targeted location, thereby quickly finding the lesion area and performing laser treatment on the skin lesion.

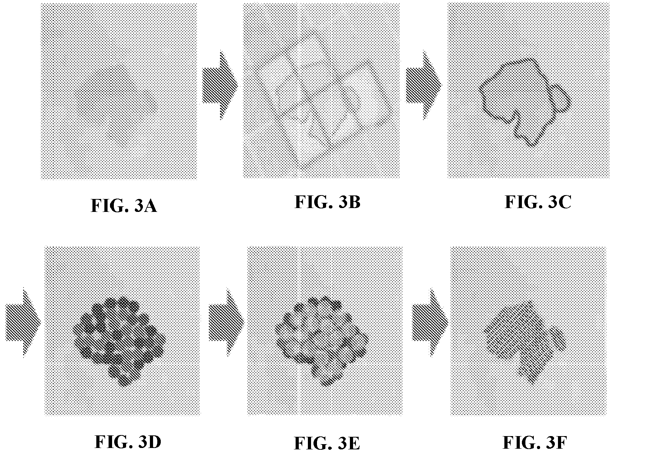

For example, it is possible to find the location of the skin lesion by analyzing the image of the contact area by the steps illustrated in FIGS. 3A-3F and to perform a laser treatment on the skin lesion using the camera of the laser device according to the embodiment illustrated in FIG. 2.

FIG. 3A is a schematic image captured by the camera as described above. The captured image can be processed and analyzed, as illustrated in FIG. 3B, to specify the boundary of the skin lesion area as illustrated in FIG. 3C. After specifying the boundary of the skin lesion area, the laser beam can be irradiated targeting the locations within the boundary, forming multiple beam spots within the boundary as illustrated in FIG. 3D. In certain embodiments, the laser beam can be irradiated in an overlapping manner between multiple beam spots as illustrated in FIG. 3E to complete the laser treatment. FIG. 3F shows a type of handling after a laser treatment.

The embodiment illustrated in FIG. 2 can be constructed such that laser beam La can be irradiated on the skin contact surface **325** after being transmitted through the beam splitter **350** from the laser transmission surface **315** of the light transmitting member **300,** and the camera **600** can capture the image from the light reflected by the beam splitter on one side of the light transmitting member **300.**

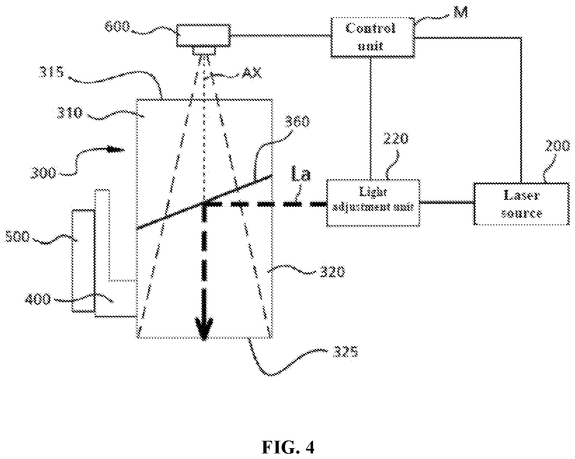

FIG. 4 illustrates another embodiment in which a laser device can be constructed to comprise: a laser source **200,** a light transmitting member **300,** cooling unit **400** and **500** in the same manner as the laser device according to the embodiment illustrated in FIGS. 1A and 1B; and in addition, comprise a light adjustment unit **220,** a beam splitter **360;** and a camera **600;** and a control unit **M** that controls these components.

The laser device according to this embodiment, as illustrated in FIG. **4****,** can be constructed such that the optical axis **AX** of the camera **600** is to be transmitted through the laser transmission surface **315** and the skin contact surface **325** of the light transmitting member **300,** thereby guiding the light reflected from the skin contact surface **325** to the camera **600,** being transmitted through the beam splitter **360** and the laser transmission surface **315.**

In the embodiment illustrated in FIG. 2 and the embodiment illustrated in FIG. 4, the locations of the laser beam and that of the camera can be swapped with each other.

In the embodiment illustrated in FIG. 4. the beam splitter **360** provided between the first light transmitting portion **310** and the second transmitting portion **320** can have different physical properties from the beam splitter **350** used in the embodiment illustrated in FIG. 2 above. The properties of the beam splitter **350** of the embodiment illustrated in FIG. 2 can allow the visible ray to be reflected and the laser beam to be transmitted. The beam splitter **360** of the embodiment illustrated in FIG. 4 can reflect the laser beam and transmit the visible light.

The light adjustment unit **220** can adjust the path of the laser beam oscillated from the laser source **200** and set the coordinates of the targeted location considering the reflection by the beam splitter **360.** The light adjustment unit **220** can be implemented with an apparatus such as a Galbano mirror.

The control unit **M** can detect the skin lesion area by performing predetermined processing and analysis of the images of the skin contact area, which the camera **600** captures via the laser transmission surface **315.** The control unit **M** can specify the boundary, calculate the location coordinates of the specified lesion within the boundary as the coordinate information of the laser beam target location, control the light adjustment unit **220** according to the coordinate information about the targeted location, and guide the laser beam **La** to be irradiated on the calculated target location, thereby quickly finding the lesion area and promptly and precisely performing laser treatment on the lesion area.

In the embodiments illustrated in FIG. 2 or FIG. 4, while the cooling unit **400** and **500** can cool down the light transmitting member **300,** the light transmitting member **300** can get foggy when a certain time elapses as the temperature of the light transmitting member falls below a certain temperature.

Fogging on the light transmitting member **300** can happen at any moment depending on the material of the light transmitting member, the ambient temperature and humidity, and the cooling temperature, among other things. When the light transmitting member **300** becomes foggy, clarity of the images captured by the camera **600** significantly degrades, making it difficult to detect the location of the lesion.

For example, in the embodiment illustrated in FIG. 2, the camera captures the image by receiving the reflected light from the beam splitter on one side of the light transmitting member **300.** As such, fogging on the lateral surface of the light transmitting member **300** can affect the clarity of the captured images. In the embodiment illustrated in FIG. 4, the camera can capture the image by receiving the light transmitted by the beam splitter **360** on the laser contact surface **315** of the light transmitting member **300.** As such, fogging on the laser transmission surface **315** of the light transmitting member **300** can affect the clarity of the captured image.

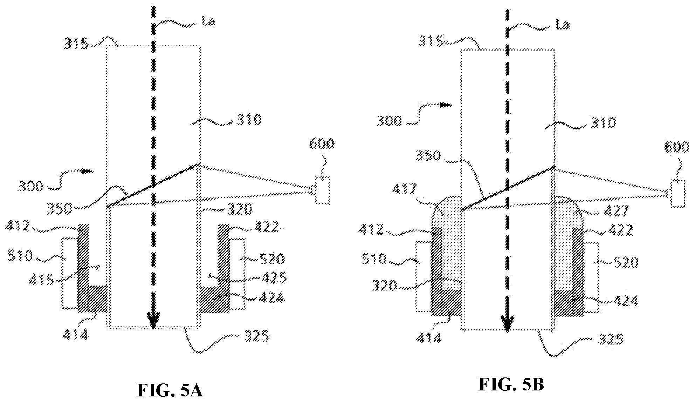

FIG. 5A and FIG. 5B illustrate exemplary embodiments to solve the fogging problem that can occur to the light transmitting member as described above. The embodiments in FIG. 5A and FIG. 5B are substantially identical to the laser device illustrated in FIG. 2 except for the elements to solve the fogging problem. The elements to solve the fogging problem on the laser device illustrated in FIG. 5A and FIG. 5B can be applied to various other embodiments, including the ones illustrated in FIGS. 1A and 1B and FIG. 4.

In FIG. 5A, the laser cooling unit according to this embodiment can comprise: thermoelectric elements **510** and **520,** and the conductive members **412, 414, 422,** and **424** for cooling down the proximate areas of the skin contact surface **325** of the light transmitting member by thermal conduction.

FIG. 5A shows the cooling unit (thermoelectric element and conductive member) located on one lateral side and another lateral side of the light transmitting member **300,** respectively, the location of the cooling unit is not limited thereto. The cooling unit can be attached only to one side of the light transmitting member, or to all sides as well.

The conductive member can comprise: (i) conductive bases **412** and **422** attached to the heat absorption plate of the thermoelectric elements **510** and **520** to conduct heat; and (ii) conductive bridges **414** and **424** for cooling down the light transmitting member **300** by conducting heat between the conductive base **412** and the light transmitting member **300.**

The conductive bridges **414** and **424** can be provided to form the spaced portions **415** and **425** between the conductive bases **412** and **422** and the light transmitting member **300.**

The distance between said spaced portions **415** and **425** can be the same as the one between the conductive bridges **414** and **424.** Preferably, the lengthwise gap of the spaced portions **415** and **425** can be longer than the length of the conductive bridges **414** and **424.** In other words, it is preferable that the volume of the spaced portions **415** and **425** is formed to be larger than the volume of the conductive bridges **414** and **424.**

Preferably, the locations to attach the conductive bridges **414** and **424** can be in the proximity of the skin contact surface **325** of the light transmitting member **300.**

In the embodiment illustrated in FIG. 5A, the conductive bases **412** and **422** attached to the thermoelectric elements **510** and **520** can only conduct heat to the conductive bridges **414** and **424,** and heat is not conducted through the spaced portions **415** and **425,** and the conductive bridges **414** and **424** are attached in the proximity of the skin contact surface **325.** As such, fogging on the light transmitting member can be significantly delayed, while substantially maintaining the effect of cooling the skin contact surface **325.**

As described above, when the thermoelectric elements **510** and **520** are connected to the light transmitting member 300 via the conductive bridges **414** and **424,** occurrence of fogging onto the light transmitting member **300** can be more significantly delayed compared to the instances where the thermoelectric elements **510** and **520** are directly attached to the light transmitting member **300.**

As illustrated in FIG. 5B, the spaced portions **415** and **425** between the conductive bases **412** and **422** and the light transmitting member **300** can be filled with the insulation materials **417** and **427,** thereby further delaying occurrence of fogging onto the light transmitting member.

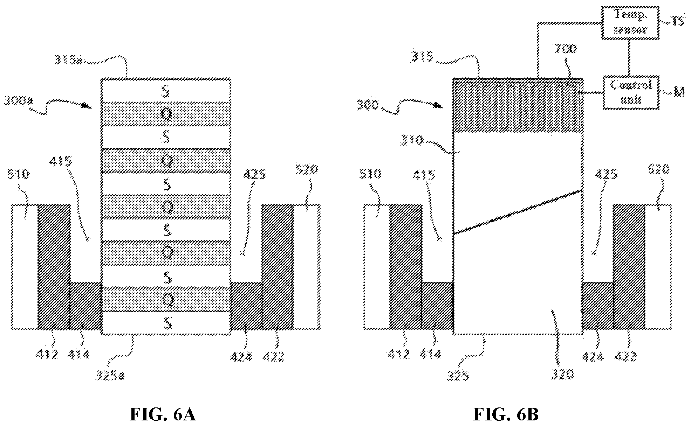

FIG. 6A illustrates yet another embodiment of the laser device in which fogging caused by cooling of the light transmitting member can be prevented or delayed by way of the construction characteristics of the laser light transmitting member.

The light transmitting member **300a** provided in the laser device according to the embodiment illustrated in FIG. 6A can be constructed by alternatingly stacking up a first light transmitting block **S** and a second light transmitting block **Q,** with two different heat transfer coefficients, from the laser transmission surface **315a** to the skin contact surface **325a,** as illustrated. Hereunder, the light transmitting member **300a** constructed by stacking up a first light transmitting block **S** and a second light transmitting block **Q** as described above is called "Stacked-up Light Transmitting Member."

In certain embodiments, aforementioned first light transmitting block **S** of Stacked-up Light Transmitting Member **300a** can be formed of, for example, a sapphire material; and the second light transmitting block **Q** can be formed of, for example, a quartz material; yet, the first light transmitting block **S** and the second light transmitting block **Q** are not limited to these materials. Insofar as the two materials have light transmission properties and mutually different thermal conductivities, any combination of materials can be used.

The thermal conductivity of sapphire is 20 W/mk and the thermal conductivity of quartz is 1.5 W/mk with a significant difference in thermal conductivity between the two materials. Thus, the heat transfer between the two blocks can be inhibited by constructing the first light transmitting block **S** and the second light transmitting block **Q** with a combination of materials with mutually different thermal conductivities and by alternatingly stacking up the blocks. As a result, fogging of Stacked-up Light Transmitting Member 300a can be significantly delayed.

The laser device according to yet another embodiment can comprise a temperature maintaining means that maintains the temperature of the light transmitting member within a set range to prevent or delay occurrence of fogging onto the light transmitting member caused by the cooling unit. FIG. 6B illustrates yet another embodiment in which a heater is attached to the light transmitting member as an example of the temperature maintaining means.

FIG. 6B illustrates an embodiment of the laser device constructed by attaching a heater **700** on one side of the light transmitting member **300.** When the light transmitting member **300** is cooled down by the cooling unit, the control unit **M** can sense the temperature of the light transmitting member **300.** Once the temperature reaches a predetermined level, it can prevent or delay occurrence of fogging onto the light transmitting member **300** by controlling the heater **700.**

In certain embodiments, as illustrated in FIG. 6B, to prevent fogging on the laser transmission surface **315** of the light transmitting member **300,** the temperature sensor **TS** can be constructed to sense the temperature of the laser transmission surface **315,** and the heater **700** can be attached in the proximity of the laser transmission surface **315.** When the temperature sensor **TS** senses the temperature of the laser transmission surface **315** and the control unit **M** determines that temperature needs to be adjusted to prevent fogging based on the sensed temperature, the temperature of the light transmitting member **300** can be adjusted by operating the heater **700** while the cooling is underway.

The heater **700** can be implemented in the form of a heat wire that generates heat, and can be implemented with a polyimide heater as well.

If a camera is positioned on the lateral surface of the light transmitting member, the surface in the direction where the camera of the light transmitting member is positioned must be free of fogging. Thus, the heater can be attached in the proximity of the surface on the side where the camera is positioned, thereby enabling the temperature control.

The method of preventing fogging with the above-referenced heater can be implemented by attaching a heater to the above-referenced light transmitting member or by attaching a heater to Stacked-up Light Transmitting Member.

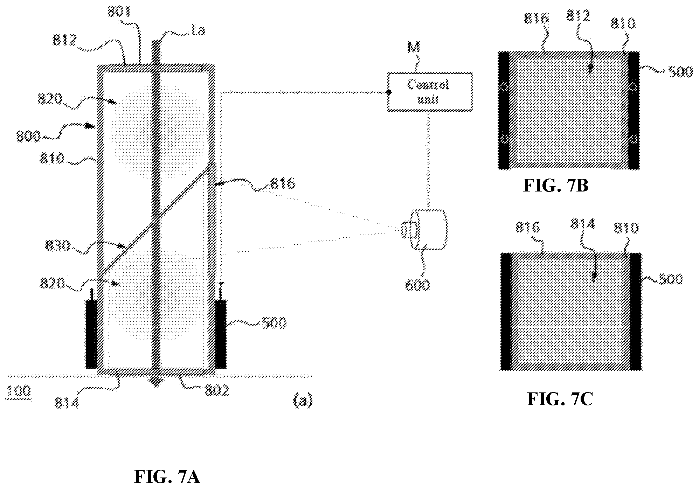

FIGS. 7A, 7B, and 7C illustrate yet another embodiment of the laser device. FIG. 7B is a plan view of the laser device illustrated in FIG. 7A. FIG. 7C is a bottom view of the laser device illustrated in FIG. 7A.

The embodiment illustrated in FIG. 7 can include a "Light Transmission Device **800"** instead of the light transmitting member, the main component of the embodiment illustrated in FIG. 2 or FIG. 4 described above. In certain embodiments, the heater **700** in FIG. 6B can be implemented in the embodiment shown in FIG. 7A in the same manner as explained above.
In certain embodiments, the implementation to solve the fogging problem on the laser device illustrated in FIG. 5A and FIG. 5B can be applied to the embodiment illustrated in FIG. 7A.

Said light transmission device **800,** as illustrated in FIG. 7A, can comprise: a conductor frame **810** formed of a conductor; a light transmission gas **820** tightly sealed inside the conductor frame **810;** a first window **812** forming a laser transmission surface **801** on the top surface of the conductor frame **810;** and a second window **814** forming a skin contact surface **802** on the bottom surface of the conductor frame **810.**

Accordingly, laser beam **La** for treatment can transmitted from the laser transmission surface **801** of the first window **812** through the light transmission gas **820** to the skin contact surface **802** of the second window **814.**

Here, a beam splitter **830** can be provided inside the conductor frame **810** and a third window **816** can be provided on one side of the conductor frame **810** so that the light from the skin contact surface **802,** reflected by the beam splitter **830,** can reach the camera **600** via the third window **816,** thereby making it possible to capture the image of the contact area of the skin contacted by the skin contact surface.

The skin contact surface **802,** the bottom surface of said second window **814,** can be constructed to contact the skin **100** for direct cooling of the contact area on the skin by the cooling unit **500.** Preferably, the second window **814** can be formed of a material that is capable of transmitting light, and yet has high thermal conductivity.

In case of above-noted embodiment, when the cooling unit **500,** e.g., thermoelectric element, operates, the second window **814** can be cooled down by heat transfer of the conductor frame **810,** and the skin contact surface **802** of the second window **814** can cool down the skin contact area and induce vasoconstriction in the proximity. When the camera **600** captures the image of the skin lesion area via third window **816** and the beam splitter **830** under such vasoconstriction, specifies the location of the skin lesion, and detects the targeted area, the laser beam can be guided to be irradiated on the targeted area, thereby performing laser treatment under vasoconstriction.

Here, the conductor frame **810** can be cooled down by the cooling unit **500.** However, since the light transmission gas **820** can be tightly sealed therein, heat transfer cannot be carried out by the gas inside. As such, fogging of each of the windows **812, 814,** and **816** can be prevented or significantly reduced or delayed.

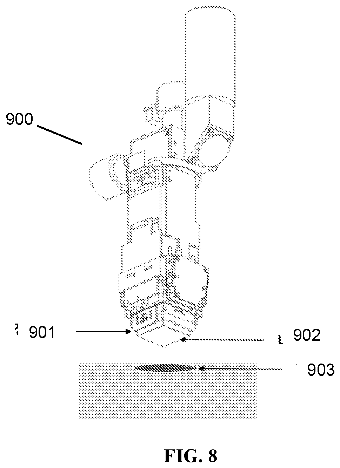

If the laser light is irradiated without a complete contact between the skin contact surface **902** of the light transmitting member and the skin lesion **903,** the laser can be irradiated on the unwanted to area of the skin through the gap between the skin contact surface 902 and the skin lesion **903,** thereby damaging the skin. FIG. 8 illustrates a hand piece of a laser device implemented with a touch sensor **901** that can detect a contact between the skin contact surface **902** of the light transmitting member and the skin lesion **903** to ensure the safety of the practitioner and the patient.

In certain embodiments, a complete contact between the skin contact surface **902** and the skin lesion **903** can be ensured by: (i) contacting the skin contact surface **902** of the light transmitting member (e.g. in a hand piece) with the treatment site **903;** (ii) measuring the impedance of the treatment site using the touch sensor; (iii) determining whether a complete contact has been made depending on the measured impedance value; and (iv) irradiating the laser only when a complete contact has been made. In this embodiment, the laser will not be irradiated when there is no complete contact.

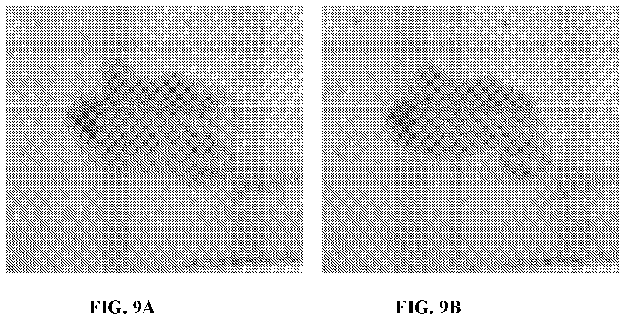

When the skin lesion is identified by a computer algorithm, there can be a mismatch between the area identified the computer algorithm (illustrated in FIG. 9A) and the area a practitioner intends to treat (illustrated in FIG. 9B). If the laser is irradiated not only to the skin lesion, but also to the normal skin unintentionally, the normal skin can suffer various adverse reactions, such as bums, wound infection, and hyperpigmentation. Therefore, it can be beneficial for a practitioner to determine the irradiation area manually when the computer algorithm fails to accurately identify the lesion area.

In certain embodiments, a scanner can facilitate irradiation of the laser beam to the treatment site based on the image obtained by the camera. In certain embodiments, the scanner can be a galvanometer scanner.

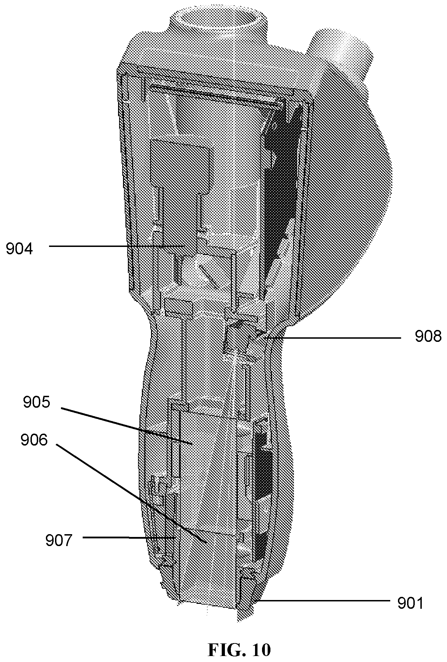

FIG. 10 shows an exemplary embodiment of a hand piece, which includes a scanner **904,** the first light transmitting portion **905,** the second light transmitting portion **906,** thermoelectric cooling modules **907,** and a camera **908.** As explained above, the first light transmitting portion **905** and the second light transmitting portion **906** can be formed of materials that can transmit light, respectively, such as quartz, sapphire, crystal, poly (methyl methacrylate), or polystyrene. The first light transmitting portion **905** and the second light transmitting portion **906** can comprise different materials from each other; and the second light transmitting portion **906** with the skin contact surface **902** can have a material with higher thermal conductivity than the first light transmitting portion **905.**

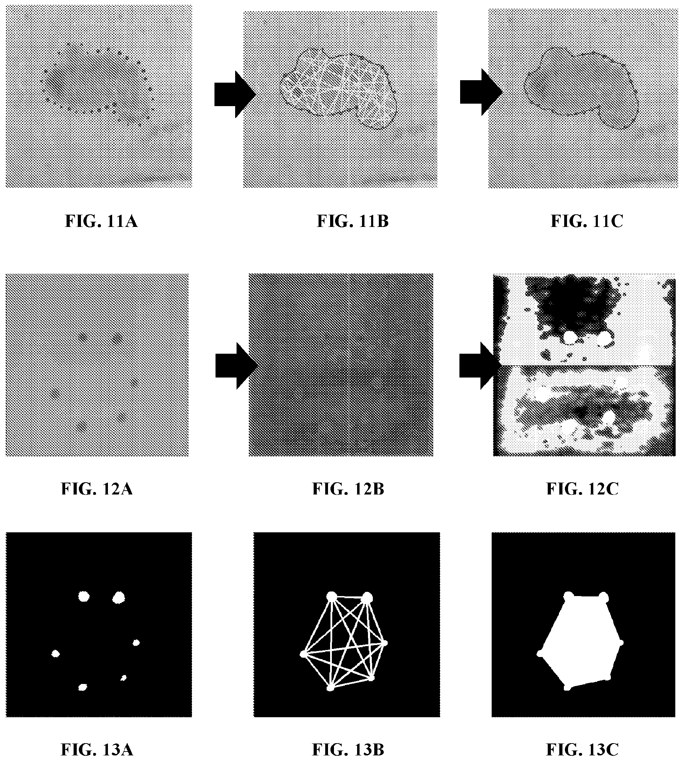

In certain embodiment, the camera **908** can be operatively coupled with a control unit to analyze characteristics of skin lesion and to guide the laser beam to treat the skin lesion according to characteristics of the skin lesions. The characteristics of the skin lesion can include location, boundary, size, thickness, color, and pigment level (i.e. pigment density or concentration) of the lesion. In certain other embodiments, the practitioner can manually select and mark the area to irradiate with dots using a pen (FIG. 11A).

An image obtained by a camera (FIG. 12A) can be transformed using a threshold image processing method to extract the area marked by the scanner (FIG. 13A). After adjusting the hue saturation value, the dots on the image transformed using the threshold image processing method can be connected (FIG. 11B). FIG. 12B shows the same image as FIG. 12A with the hue saturation value. FIG. 13B shows the same image as FIG. 13A with the threshold hue saturation value. Using an equalized histogram, the internal area connected by dots can be filled and the laser irradiation area can be designated by adjusting the threshold value (FIG. 11C). Finally, the designated area can be irradiated with the laser. FIG. 12C shows the same image as FIG. 12A with an area filled using an equalized histogram. FIG. 13C shows the same image as FIG. 13A with an area filled using an threshold equalized histogram.

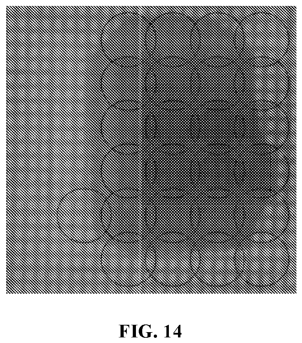

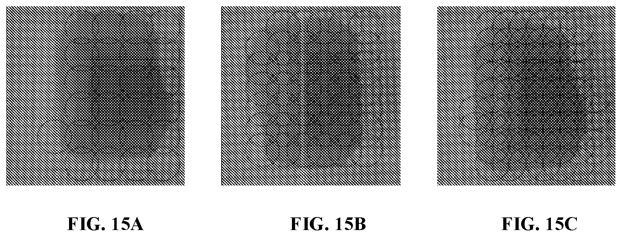

When irradiating the laser light to an identified target skin lesion, there can be an area where the laser light is not irradiated depending on the size of the laser spot (FIG. 14). To ensure the laser light is irradiated on the entire skin lesion, an overlapping percentage value can be designated depending on the size of the laser spot so that the laser can be irradiated in an overlapping manner accordingly. FIGS. 15A, 15B, and 15C show exemplary irradiation patterns when the overlapping percentage values are 20%, 40%, and 50%, respectively. The irradiation pattern can be determined according to the laser spot size (mm), the overlap percentage value (%), treatment area (mm²), and resolution of the image obtained by the camera (pixel). By using the laser device disclosed herein, the skin lesion can be treated with laser light under vasoconstriction so that damages caused by the laser energy, especially in the overlapping area, can be minimized even if higher than conventional fluence is irradiated on the overlapping area.

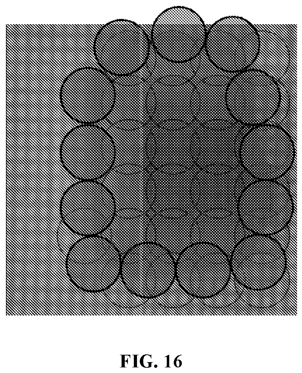

In a pigmented lesion, pigments can be more widely distributed underneath the surface of the skin than what is seen on the skin surface. As such, it may not be sufficient to treat the entire volume of a skin lesion if the laser light is irradiated on the area identified on the skin surface. To thoroughly treat a skin lesion underneath the skin surface, the laser light can be irradiated on a normal skin area surrounding the skin lesion beyond the boundary identified by a computer algorithm or manually by a practitioner on the skin surface. In certain circumstances, the center of the laser spot can be targeted to the boundary of the skin lesion so that the periphery of the laser spot reaches beyond the boundary of the skin lesion on the skin surface (FIG. 16). In certain other circumstances, the center of the laser spot can be targeted beyond the boundary of the skin lesion identified on the skin surface. The skin lesion can be treated with laser light under vasoconstriction so that damages caused by the laser energy can be minimized for the treated area.

Other embodiments are within the scope of the following claims.

## Claims

1. A device for treating a skin lesion (110)comprising:
a laser source (200);
a light transmitting member (300), which comprises a top surface that forms a laser transmission surface (315) and a bottom surface that forms a skin contacting surface (325), wherein the light transmitting member (300) allows a treatment laser beam from the laser source (200) to transmit from the laser transmission surface (315) to the skin contacting surface (325);
a cooling unit (400,500) configured to cause the skin (100) contacting surface (325) of the light transmitting member (300) to cool, thereby inducing vasoconstriction of a treatment site by cooling the treatment site with the skin contacting surface (325);
a control unit (M) configured to control the cooling unit and enable the treatment laser beam to be irradiated on the skin lesion (110) in the treatment site under vasoconstriction by controlling the laser source (200); and
a heater (700) attached to the light transmitting member, wherein the control unit (M) is configured to control the heater (700) to prevent or delay fogging on the light transmitting member (300),
wherein the light transmitting member (300) comprises a first light transmitting portion (310) and a second light transmitting portion (320),
wherein the second light transmitting portion (320) has a higher thermal conductivity than the first light transmitting portion (310), **characterized in that**
the first light transmitting portion (310) is in contact with the second light transmitting portion (320).

2. The device of claim 1, wherein the first light transmitting portion (310) and the second light transmitting portion (320) are optically transparent.

3. The device of claim 2, wherein the first light transmitting portion (310) and/or the second light transmitting portion (320) includes quartz, sapphire, crystal, poly(methyl methacrylate), or polystyrene.

4. The device of claim 2, further comprising a beam splitter located between the first light transmitting portion (310) and the second light transmitting portion (320).

5. The device of claim 1, wherein the cooling unit (400,500) includes a thermoelectric element (500), cooling air, cooling gas, or cooling liquid.

6. The device of claim l, wherein the cooling unit (400,500) comprises a thermoelectric element (500) and a conductive member (400), preferably wherein the thermoelectric element (500) is attached to one side of the light transmitting member, more preferably wherein the thermoelectric element (500) is attached to one side of the light transmitting member (300) and also attached to the second light transmitting portion (320).

7. The device of claim 6, wherein the conductive member (400) is placed between the thermoelectric element (500) and the light emitting member.

8. The device of claim 7, wherein the thermoelectric element (500) comprises a heat absorption plate and a heat radiation plate contacting each other.

9. The device of claim 8, wherein the conductive member (400) comprises a conductive base attached to the heat absorption plate of the thermoelectric element (500), preferably further comprising an insulation material placed between the conductive base and the light transmitting portion.

10. The device of claim 8, wherein the conductive member (400) comprises a conductive bridge placed between the thermoelectric element (500) and the light emitting element.

11. The device of claim l, further comprising a temperature sensor, preferably wherein the temperature sensor is configured to detect the temperature of the light emitting member, the cooling unit (400,500) and/or the treatment site.

12. The device of claim l, further comprising a camera configured to obtain an image of the treatment site that comes into contact with the skin contacting surface (325).

13. The device of claim 1,, wherein the first light transmitting portion and the second light transmitting portion are alternatingly stacked up from the laser transmission surface to the skin contacting surface (325).

14. The device of claim l, further comprising a sensor configured to detect a gap between the skin contacting surface (325) and the treatment site.

15. The device of claim 12, further comprising a scanner that facilitates irradiation of the laser beam to the treatment site based on the image obtained by the camera.

## Patentansprüche

1. Eine Vorrichtung zur Behandlung einer Hautläsion (110), die folgende Merkmale aufweist:
eine Laserquelle (200);
ein lichtübertragendes Bauglied (300), das eine obere Oberfläche, die eine Laserübertragungsoberfläche (315) bildet, und eine untere Oberfläche aufweist, die eine Haut-kontaktierende Oberfläche (325) bildet, wobei das lichtübertragende Bauglied (300) es ermöglicht, dass ein Behandlungslaserstrahl aus der Laserquelle (200) von der Laserübertragungsoberfläche (315) zu der Haut-kontaktierenden Oberfläche (325) übertragen wird;
eine Kühleinheit (400, 500), die dazu konfiguriert ist, zu bewirken, dass die Haut-(100)-kontaktierende Oberfläche (325) des lichtübertragenden Bauglieds (300) sich abkühlt, wodurch eine Vasokonstriktion einer Behandlungsstelle ausgelöst wird, indem die Behandlungsstelle mit der Haut-kontaktierenden Oberfläche (325) gekühlt wird;
eine Steuereinheit (M), die dazu konfiguriert ist, die Kühleinheit zu steuern und es zu ermöglichen, dass der Behandlungslaserstrahl auf die Hautläsion (110) an der Behandlungsstelle bei Vasokonstriktion gestrahlt wird, indem die Laserquelle (200) gesteuert wird; und
eine Wärmevorrichtung (700), die an dem lichtübertragenden Element angebracht ist, wobei die Steuereinheit (M) dazu konfiguriert ist, die Wärmevorrichtung (700) zu steuern, um ein Beschlagen des lichtübertragenden Bauglieds (300) zu verhindern oder zu verzögern,
wobei das lichtübertragende Bauglied (300) einen ersten lichtübertragenden Abschnitt (310) und einen zweiten lichtübertragenden Abschnitt (320) aufweist, wobei der zweite lichtübertragende Abschnitt (320) eine höhere thermische Leitfähigkeit als der erste lichtübertragende Abschnitt (310) aufweist, **dadurch gekennzeichnet, dass**
der erste lichtübertragende Abschnitt (310) mit dem zweiten lichtübertragenden Abschnitt (320) in Kontakt steht.

2. Die Vorrichtung gemäß Anspruch 1, wobei der erste lichtübertragende Abschnitt (310) und der zweite lichtübertragende Abschnitt (320) optisch transparent sind.

3. Die Vorrichtung gemäß Anspruch 2, wobei der erste lichtübertragende Abschnitt (310) und/oder der zweite lichtübertragende Abschnitt (320) Quarz, Saphir, Kristall, Polymethylmethacrylat oder Polystyrol umfassen.

4. Die Vorrichtung gemäß Anspruch 2, die ferner einen Strahlteiler aufweist, der sich zwischen dem ersten lichtübertragenden Abschnitt (310) und dem zweiten lichtübertragenden Abschnitt (320) befindet.

5. Die Vorrichtung gemäß Anspruch 1, wobei die Kühleinheit (400, 500) ein thermoelektrisches Element (500), Kühlluft, Kühlgas oder Kühlflüssigkeit umfasst.

6. Die Vorrichtung gemäß Anspruch 1, wobei die Kühleinheit (400, 500) ein thermoelektrisches Element (500) und ein leitfähiges Bauglied (400) aufweist, wobei vorzugsweise das thermoelektrische Element (500) an einer Seite des lichtübertragenden Elements angebracht ist, wobei besonders vorzugsweise das thermoelektrische Element (500) an einer Seite des lichtübertragenden Bauglieds (300) angebracht ist und auch am zweiten lichtübertragenden Abschnitt (320) angebracht ist.

7. Die Vorrichtung gemäß Anspruch 6, wobei das leitfähige Bauglied (400) zwischen dem thermoelektrischen Element (500) und dem lichtemittierenden Bauglied platziert ist.

8. Die Vorrichtung gemäß Anspruch 7, wobei das thermoelektrische Element (500) eine Wärmeabsorptionsplatte und eine Wärmestrahlungsplatte aufweist, die sich gegenseitig kontaktieren.

9. Die Vorrichtung gemäß Anspruch 8, wobei das leitfähige Bauglied (400) eine leitfähige Basis aufweist, die an die Wärmeabsorptionsplatte des thermoelektrischen Elements (500) angebracht ist, wobei die Vorrichtung vorzugsweise ferner ein Isolationsmaterial, das zwischen der leitfähigen Basis und dem lichtübertragenden Abschnitt platziert ist, aufweist.

10. Die Vorrichtung gemäß Anspruch 8, wobei das leitfähige Bauglied (400) eine leitfähige Brücke aufweist, die zwischen dem thermoelektrischen Element (500) und dem lichtemittierenden Element platziert ist.

11. Die Vorrichtung gemäß Anspruch 1, die ferner einen Temperatursensor aufweist, wobei vorzugsweise der Temperatursensor dazu konfiguriert ist, die Temperatur des lichtemittierenden Bauglieds, der Kühleinheit (400, 500) und/oder der Behandlungsstelle zu detektieren.

12. Die Vorrichtung gemäß Anspruch 1, die ferner eine Kamera aufweist, die dazu konfiguriert ist, ein Bild der Behandlungsstelle zu erhalten, die mit der Haut-kontaktierenden Oberfläche (325) in Kontakt tritt.

13. Die Vorrichtung gemäß Anspruch 1, wobei der erste lichtübertragende Abschnitt und der zweite lichtübertragende Abschnitt im Wechsel von der Laserübertragungsoberfläche zu der Haut-kontaktierenden Oberfläche (325) gestapelt sind.

14. Die Vorrichtung gemäß Anspruch 1, die ferner einen Sensor aufweist, der dazu konfiguriert ist, eine Lücke zwischen der Haut-kontaktierenden Oberfläche (325) und der Behandlungsstelle zu detektieren.

15. Die Vorrichtung gemäß Anspruch 12, die ferner einen Scanner aufweist, der eine Strahlung des Laserstrahl auf die Behandlungsstelle ermöglicht, basierend auf dem Bild, das von der Kamera erhalten wird.

## Revendications

1. Dispositif pour traiter une lésion cutanée (110) comprenant :
une source laser (200) ;
un élément transmettant la lumière (300), qui comprend une surface supérieure qui forme une surface de transmission laser (315) et une surface inférieure qui forme une surface en contact avec la peau (325), dans lequel l'élément transmettant la lumière (300) permet à un faisceau laser de traitement provenant de la source laser (200) de passer de la surface de transmission laser (315) à la surface en contact avec la peau (325) ;
une unité de refroidissement (400,500) configurée pour refroidir la surface (325) de l'élément transmettant la lumière (300) en contact avec la peau (100), induisant ainsi une vasoconstriction d'un site de traitement en refroidissant le site de traitement avec la surface (325) en contact avec la peau ;
une unité de commande (M) configurée pour commander l'unité de refroidissement et permettre au faisceau laser de traitement d'être irradié sur la lésion cutanée (110) dans le site de traitement sous vasoconstriction en commandant la source laser (200) ; et
un dispositif de chauffage (700) fixé à l'élément transmettant la lumière, dans lequel l'unité de commande (M) est configurée pour commander le dispositif de chauffage (700) afin d'empêcher ou de retarder la formation de buée sur l'élément transmettant la lumière (300),
dans lequel l'élément transmettant la lumière (300) comprend une première partie transmettant la lumière (310) et une deuxième partie transmettant la lumière (320),
dans lequel la deuxième partie transmettant la lumière (320) a une conductivité thermique supérieure à celle de la première partie transmettant la lumière (310), **caractérisé en ce que** la première partie transmettant la lumière (310) est en contact avec la deuxième partie transmettant la lumière (320).

2. Dispositif selon la revendication 1, dans lequel la première partie transmettant la lumière (310) et la deuxième partie transmettant la lumière (320) sont optiquement transparentes.

3. Dispositif selon la revendication 2, dans lequel la première partie transmettant la lumière (310) et/ou la deuxième partie transmettant la lumière (320) comprend du quartz, du saphir, du cristal, du poly(méthacrylate de méthyle) ou du polystyrène.

4. Dispositif selon la revendication 2, comprenant en outre un séparateur de faisceau situé entre la première partie transmettant la lumière (310) et la deuxième partie transmettant la lumière (320).

5. Dispositif selon la revendication 1, dans lequel l'unité de refroidissement (400,500) comprend un élément thermoélectrique (500), de l'air de refroidissement, un gaz de refroidissement ou un liquide de refroidissement.

6. Dispositif selon la revendication 1, dans lequel l'unité de refroidissement (400,500) comprend un élément thermoélectrique (500) et un élément conducteur (400), de préférence dans lequel l'élément thermoélectrique (500) est fixé à un côté de l'élément transmettant la lumière, plus préférablement dans lequel l'élément thermoélectrique (500) est fixé à un côté de l'élément transmettant la lumière (300) et également fixé à la deuxième partie transmettant la lumière (320).

7. Dispositif selon la revendication 6, dans lequel l'élément conducteur (400) est placé entre l'élément thermoélectrique (500) et l'élément émetteur de lumière.

8. Dispositif selon la revendication 7, dans lequel l'élément thermoélectrique (500) comprend une plaque d'absorption de chaleur et une plaque de rayonnement de chaleur en contact l'une avec l'autre.

9. Dispositif selon la revendication 8, dans lequel l'élément conducteur (400) comprend une base conductrice fixée à la plaque d'absorption de chaleur de l'élément thermoélectrique (500), comprenant de préférence en outre un matériau isolant placé entre la base conductrice et la partie transmettant la lumière.

10. Dispositif selon la revendication 8, dans lequel l'élément conducteur (400) comprend un pont conducteur placé entre l'élément thermoélectrique (500) et l'élément émetteur de lumière.

11. Dispositif selon la revendication 1, comprenant en outre un capteur de température, de préférence dans lequel le capteur de température est configuré pour détecter la température de l'élément émetteur de lumière, de l'unité de refroidissement (400,500) et/ou du site de traitement.

12. Dispositif selon la revendication 1, comprenant en outre une caméra configurée pour obtenir une image du site de traitement qui entre en contact avec la surface en contact avec la peau (325).

13. Dispositif selon la revendication 1, dans lequel la première partie transmettant la lumière et la deuxième partie transmettant la lumière sont empilées en alternance depuis la surface de transmission laser jusqu'à la surface en contact avec la peau (325).

14. Dispositif selon la revendication 1, comprenant en outre un capteur configuré pour détecter un espace entre la surface en contact avec la peau (325) et le site de traitement.

15. Dispositif selon la revendication 12, comprenant en outre un scanner qui facilite l'irradiation du faisceau laser vers le site de traitement sur la base de l'image obtenue par la caméra.
